# FASCICULE DE BREVET EUROPEEN

(11) **EP 2 478 112 B1**
(45) Date de publication et mention de la délivrance du brevet: **26.06.2013**
(21) Numéro de dépôt: 10770572.5
(22) Date de dépôt: 16.09.2010
(51) Int. Cl.: C12Q 1/04, C12Q 1/44

(54) **PROCEDE D'IDENTIFICATION DE BACTERIES DU GROUPE BACILLUS CEREUS**
VERFAHREN ZUR IDENTIFIZIERUNG VON BAKTERIEN DER GRUPPE BACILLUS CEREUS
METHOD FOR IDENTIFYING BACTERIA FROM THE BACILLUS CEREUS GROUP

(30) Priorité: 18.09.2009 FR 0904470
(43) Date de publication de la demande: 25.07.2012
(73) Titulaire: Biomérieux, 69280 Marcy L'etoile (FR)
(72) Inventeur: CELLIER, Marie, F-38390 Montalieu Vercieu (FR); MILLS, John, Fenton Missouri 63026 (US); MOSTICONE, David, F-69280 Sainte Consorce (FR); ORENGA, Sylvain, F-01160 Neuville Sur Ain (FR); VIMONT, Antoine, F-69005 Lyon (FR)
(86) Numéro de dépôt international: PCT/FR2010/051925
(87) Numéro de publication internationale: WO 2011/033224

(56) Documents cités:
- EP-A1- 1 219 628
- US-A1- 2004 005 652
- US-B1- 6 284 517
- JUERGENSMEYER MARGARET A ET AL: "A selective chromogenic agar that distinguishes Bacillus anthracis from Bacillus cereus and Bacillus thuringiensis.", JOURNAL OF FOOD PROTECTION AUG 2006 LNKD- PUBMED:16924932, vol. 69, no. 8, août 2006 (2006-08), pages 2002-2006, XP009132128, ISSN: 0362-028X
- FRICKER ET AL: "Evaluation of standard and new chromogenic selective plating media for isolation and identification of Bacillus cereus", INTERNATIONAL JOURNAL OF FOOD MICROBIOLOGY, ELSEVIER SCIENCE PUBLISHERS, AMSTERDAM, NL, vol. 121, no. 1, 4 novembre 2007 (2007-11-04), pages 27-34, XP022393052, ISSN: 0168-1605
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. 55, no. 3, 1 septembre 1991 (1991-09-01), pages 335-348, XP009020939, ISSN: 0146-0749

## Description

La présente invention est relative au domaine du contrôle microbiologique alimentaire. Plus particulièrement, elle porte sur une méthode de discrimination de bactéries du groupe *Bacillus cereus* par rapport à d'autres bactéries, par hydrolyse d'un substrat fluorescent de la lécithinase ou phosphatidylcholine phospholipase C, ci-après dénommée PC-PLC.

Le contrôle microbiologique dans le domaine agro-alimentaire nécessite la mise en oeuvre de techniques qui permettent la détection et/ou l'identification et/ou le dénombrement de microorganismes, et dont le rendu de résultat doit être le plus rapide possible. Lesdits microorganismes peuvent être non pathogènes, tels les bactéries d'intérêt technologique comme les ferments ou tels des indicateurs qualité ; ces derniers permettent de valider le processus de production, des matières premières ou produits bruts, jusqu'aux produits finis. Néanmoins, lesdits microorganismes sont le plus souvent pathogènes et la détection rapide et précise de contaminations présumées permet d'engager des actions correctives. Alternativement, les toxines produites par lesdits microorganismes et responsables des effets pathogènes, peuvent être recherchées.

Le diagnostic clinique utilise les mêmes techniques : soit la détection et/ou le dénombrement des bactéries elles-mêmes, soit la détection des toxines. Dans tous les cas, les facteurs importants pour les tests diagnostiques sont : la sensibilité, la sélectivité et le temps d'obtention du résultat.

Le genre *Bacillus* comporte des bactéries à Gram positif présentes dans la nature de façon ubiquitaire : dans la terre, l'eau, l'air, ainsi que dans les produits alimentaires, des graines de céréales aux laits en poudre, produits farineux, épices. La capacité à former des spores leur confère une très grande résistance dans le milieu extérieur. Les spores de *Bacillus cereus (B. cereus),* en particulier, peuvent souiller les aliments, des matières premières aux produits manufacturés. La survie desdites spores est assurée tout au long de la chaîne alimentaire. Dans des circonstances normales, *B. cereus* est présent en quantité inférieure à 10³ cellules par gramme d'aliment et n'a aucune incidence pathogène. Le niveau minimum pathogène est supérieur à 10⁵ cellules par gramme d'aliment. La contamination d'un individu à partir d'un aliment peut alors être responsable d'une gastro-entérite. Les gastro-entérites associées à *B. cereus* se traduisent soit par des vomissements, soit par des diarrhées. Divers aliments peuvent être incriminés : viandes, riz, plat déshydratés, sauces, soupes etc. Des infections opportunistes à B. cereus peuvent aussi être observées chez des patients fragilisés tels des sujets alcooliques, immunodéprimés ou à la suite de blessures telles des brûlures.

La détection et la quantification des bactéries du groupe *Bacillus cereus* est donc primordiale pour les laboratoires de contrôle de l'industrie agro-alimentaire et pour les laboratoires de diagnostic clinique. De façon standard, l'isolement est effectué sur des milieux de culture sélectifs conventionnels sur boite : par exemple, les normes de type ISO (International Organization for Standardization) ou les méthodes BAM-FDA (Bacteriological Analytical Manual of the Food and Drug Administration) recommandent les milieux tels le polymyxin egg yolk mannitol bromothymol blue Agar (PEMBA) ou le mannitol-egg yolk-polymyxin Agar (MYP), selon leurs appellations anglo-saxonnes. L'identification est effectuée selon des caractères morphologiques et des caractères culturaux ou métaboliques.

Ces milieux PEMBA ou MYP peuvent mener à des faux positifs potentiels liés à un système inhibiteur peu performant ou à des faux négatifs, liés à l'absence possible des caractéristiques-clés morphologiques ou métaboliques chez certaines souches. Enfin, certaines souches présentent des réactions ambiguës, comme le décrit Fricker et al., International Journal of Food Microbiology ; 121 (2008) : 27-34. Des milieux chromogènes en boite ont été développés pour pallier les faux négatifs : ils contiennent des substrats naturels ou des substrats synthétiques chromogènes. Des activités enzymatiques spécifiques de certaines souches bactériennes sont ainsi détectées par le clivage de ces substrats. La spécificité de la détection peut être améliorée par l'addition dans le milieu de culture de systèmes inhibiteurs, cocktails d'antimicrobiens et/ou d'antifongiques destinés à limiter la croissance d'autres microorganismes. Cependant, les cocktails d'inhibiteurs retardent également la croissance des microorganismes cibles, dans la mesure où ils sont destinés à limiter la croissance de microorganismes du même genre que lesdits microorganismes cibles.

Des milieux chromogènes ou fluorescents, basés sur la détection de l'activité Phosphatidylinositol-specific phospholipase C (ci-après notée PI-PLC), ont été décrits dans les brevets US 6,284,517 B, EP 1 219 628 B et US 6,558,917 B, ainsi que dans la publication de Fricker *et al.,* 2008 *(supra).* Ces milieux présentent l'inconvénient de mener à des faux négatifs, en particulier avec certaines souches du groupe *Bacillus cereus* ne présentant pas d'activité PI-PLC *(B. cereus, B. mycoides, B. weihenstephanensis)* ou une faible activité PI-PLC *(B. anthracis*)*,* ou à des faux positifs. En outre, le substrat fluorescent 4MU-MIP (4-methylumbelliferyl myo-inositol-1-phosphate) présente une stabilité réduite en milieu aqueux qui impose des conditions draconiennes d'utilisation, en particulier une mesure discontinue de la fluorescence, et ce, dans des conditions de pH précises, comme l'indique le brevet US 6,558,917. Plus précisément, il est nécessaire d'effectuer la culture à pH acide puis d'alcaliniser le milieu pour augmenter la fluorescence, lue en point final. Le brevet US 7,309,580 B2 décrit un milieu en boite combinant un substrat chromogène de PC-PLC et un substrat chromogène de PI-PLC. Les couleurs respectives, du milieu, du premier substrat et du second substrat sont différentes et peuvent également se différencier de la troisième couleur résultant du mélange éventuel des produits de réactions enzymatiques.

Le milieu BCM fourni par Biosynth® AG (Suisse) utilise un substrat chromogène de PI-PLC et un système inhibiteur de la flore bactérienne non ciblée comprenant de la polymyxine B, du trimethoprime, du sulfamethoxazole et du cycloheximide. Les performances du test sont améliorées par rapport aux milieux standards mais certaines souches atypiques peuvent rester mal identifiées (Fricker *et al.,* 2008, *supra).*

Des milieux chromogènes basés sur l'hydrolyse de substrats de β-glucosidase existent, tel le Brilliance™ *Bacillus cereus* Agar fourni par Oxoid™. Celui-ci génère cependant des faux-positifs Gram positifs malgré la présence d'un système inhibiteur anti-Gram positifs comprenant de la polymyxine B et du trimethoprime, ainsi que des faux négatifs (Fricker *et al.,* 2008).

Enfin, un milieu chromogène en boite, basé sur l'hydrolyse d'un substrat chromogène de PC-PLC existe : R&F® *Anthracis* Chromogenic Agar. Celui-ci fournit une indication de résultat négatif pour *Bacillus anthracis* en 24 heures et un résultat potentiellement positif en 48 heures. Le temps d'obtention du résultat reste long et la spécificité nécessite la présence de plusieurs antibiotiques. Juergensmeyer et al., Journal of food protection ; vol. 69, No 8 (2006) : 2002-2006, ainsi que le brevet US 2004/005652 décrivent également l'utilisation de tels milieux chromogènes.

Il ressort de cet état des lieux qu'il n'existe pas, à ce jour, de procédé permettant de détecter et/ou dénombrer des bactéries du groupe *Bacillus cereus* à l'aide d'un milieu réactionnel comprenant un inhibiteur des bactéries Gram négatives et un substrat fluorescent de PC-PLC, ledit milieu réactionnel permettant d'obtenir un résultat en 6 à 30 heures. Un tel procédé présente une réelle valeur ajoutée pour le diagnostic clinique ou industriel, en particulier dans l'industrie agro-alimentaire.

Eu égard aux inconvénients relevés dans l'état de la technique considéré *supra,* les objectifs essentiels de la présente invention sont de :
● obtenir un résultat positif plus rapidement que les tests existants ;
● réduire le nombre de faux-positifs ;
● améliorer la sensibilité, en particulier pour de faibles niveaux de contamination de l'échantillon, grâce à l'utilisation d'un système inhibiteur réduit ;
● présenter une grande facilité de lecture et d'interprétation grâce à l'utilisation d'un seul substrat spécifique, ladite lecture pouvant en outre être automatisée ;
● présenter des conditions de réaction et/ou de culture simplifiées, en particulier pour la préparation du milieu et la stabilité du substrat.

Selon un premier mode de réalisation, l'invention porte sur un procédé d'identification des bactéries du groupe *Bacillus cereus,* comprenant les étapes consistant à :
● mettre en contact, dans un conteneur, un échantillon susceptible de contenir des bactéries du groupe *Bacillus cereus,* un milieu réactionnel comprenant au moins un substrat fluorescent de PC-PLC et un inhibiteur des bactéries Gram négatives;
● incuber l'ensemble ;
● identifier les bactéries du groupe *Bacillus cereus* par détection de la réaction d'hydrolyse du substrat de PC-PLC,
dans lequel le pH du milieu réactionnel et le temps nécessaire à la détection de la réaction d'hydrolyse du substrat de PC-PLC mise en oeuvre sont adaptés de telle façon que ladite réaction d'hydrolyse par les bactéries du groupe *Bacillus cereus* est détectée avant que l'hydrolyse du substrat de PC-PLC par les bactéries Gram positives autres que celles appartenant au groupe *Bacillus cereus* et potentiellement présentes dans l'échantillon, ne soit détectable.

On entend par « temps nécessaire à la détection de l'hydrolyse du substrat » le temps qui s'écoule entre la mise en contact, dans un conteneur, de l'échantillon, du milieu réactionnel et du substrat, et la détection du signal. Concrètement, ce temps est fixé et peut être assimilé au temps de réaction. Ledit signal peut alors être considéré comme un résultat. Par « avant que l'hydrolyse du substrat de PC-PLC par les bactéries Gram positives autres que celles appartenant au groupe *Bacillus cereus* et potentiellement présentes dans l'échantillon, ne soit détectable », on entend : avant l'atteinte, en l'absence de bactéries du groupe *Bacillus cereus,* d'un signal correspondant au seuil de détection desdites bactéries du groupe *Bacillus cereus.* Le résultat est considéré comme négatif si le signal obtenu n'est pas significativement différent du bruit de fond, autrement dit s'il ne dépasse pas le seuil de détection. Ledit résultat est positif si le signal obtenu est significativement différent du bruit de fond.

De façon préférentielle, le pH du milieu réactionnel est compris entre 6,8 et 8,0.

De façon préférentielle, le temps nécessaire à la détection de l'hydrolyse du substrat de PC-PLC est compris entre 6 et 30 heures.

Le milieu réactionnel mis en oeuvre dans le procédé selon l'invention est préférentiellement sous forme solide, liquide ou gel.

Avantageusement, le milieu réactionnel mis en oeuvre dans le procédé selon l'invention peut être un milieu de culture.

Avantageusement, un dénombrement des bactéries du groupe *Bacillus cereus* présentes dans l'échantillon est également possible.

Selon un mode particulier de réalisation, le procédé selon l'invention peut être mis en oeuvre dans des microplaques, des microcupules, des microtubes, des capillaires ou des cartes multipuits telles les cartes VITEK® ou TEMPO® développées et commercialisées par la Demanderesse. Avantageusement, le procédé selon l'invention peut être associé à un dispositif automatique de contrôle microbiologique de type TEMPO® tel que développé et commercialisé par la Demanderesse.

Selon un autre mode de réalisation, le procédé selon l'invention comprend en outre une étape préalable de pré-enrichissement.

Avantageusement, le milieu réactionnel mis en oeuvre dans le procédé selon l'invention comprend en outre au moins un second substrat, chromogène ou fluorescent. Selon un mode de réalisation particulier, ledit substrat est un substrat de PI-PLC, permettant de discriminer *Bacillus anthracis* de *Bacillus cereus* et *Bacillus thuringiensis.*

De façon préférentielle, le substrat fluorescent de PC-PLC correspond au 4 MU-CP (4-méthyl-umbelliferyl-choline phosphate).

Selon un mode particulier de réalisation du procédé selon l'invention, les bactéries du groupe *Bacillus cereus* sont choisies parmi *Bacillus cereus, Bacillus anthracis, Bacillus thuringiensis, Bacillus mycoides, Bacillus pseudomycoides, Bacillus weihenstephanensis,* et les autres bactéries sont choisies parmi *Listeria monocytogenes, Listeria ivanovii, Staphylococcus* ou les autres espèces du genre *Bacillus spp.* telles *Bacillus subtilis, Bacillus sphaericus, Bacillus circulans, Bacillus lentus, Bacillus pumilus, Bacillus megaterium, Paenibacillus polymyxa.*

Le procédé, objet de l'invention, peut être mis en oeuvre à l'aide d'un kit comprenant : un milieu réactionnel contenant au moins un inhibiteur des bactéries Gram négatives et un substrat fluorescent spécifique de PC-PLC. Ledit milieu est remis en suspension par un aliquot de l'échantillon à analyser. Avantageusement, le kit permettant de mettre en oeuvre le procédé selon l'invention peut aussi contenir un conteneur solide de type microplaque, microtube, microcupule, capillaire, carte multipuit telle que carte VITEK® ou carte TEMPO®. De façon préférentielle, le substrat fluorescent de PC-PLC utilisé dans le kit correspond au 4-methyl-umbiliferyl-choline phosphate (4 MU-CP). Avantageusement, le kit permettant la mise en oeuvre du procédé selon l'invention comprend en outre au moins un substrat supplémentaire, chromogène ou fluorescent. Préférentiellement, ledit substrat est un substrat de PI-PLC permettant de discriminer *Bacillus anthracis* de *Bacillus cereus, Bacillus thuringiensis, Bacillus weihenstephanensis, Bacillus mycoides* et *Bacillus pseudomycoides.*

On définit la spécificité d'un test par la combinaison de la sensibilité et de la sélectivité. La sensibilité est définie comme le pouvoir de mettre en évidence l'espèce recherchée, lorsque celle-ci est présente en faible quantité dans un échantillon à tester. Une faible sensibilité se traduira par des résultats faussement négatifs. La sélectivité, définie comme le pouvoir de détecter l'espèce recherchée dans l'échantillon contenant également d'autres espèces. L'utilisation de substrats spécifiques du métabolisme de l'espèce recherchée améliore la sélectivité. Mais des souches exprimant peu l'activité enzymatique recherchée peuvent n'être pas détectées et mener à des résultats faussement négatifs. De même, l'utilisation de cocktails d'inhibiteurs, autrement dit de composés susceptibles de ralentir, limiter ou bloquer la croissance d'espèces potentiellement présentes mais dont la détection n'est pas souhaitée, améliore la sélectivité. Un système inhibiteur peu performant peut mener à des résultats faussement positifs.

On entend par échantillon une petite partie ou petite quantité isolée d'une entité pour l'analyse. L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique), un prélèvement tissulaire ou des cellules isolées. Ce prélèvement peut être utilisé tel quel ou, préalablement à l'analyse, subir une préparation de type enrichissement, extraction, concentration, purification, selon des méthodes connues de l'homme du métier.

Le contrôle microbiologique correspond à l'analyse d'un échantillon dans le but d'isoler et/ou d'identifier et/ou d'énumérer des microorganismes potentiellement présents tels des bactéries ou des levures. Par milieu réactionnel, on entend un milieu comprenant tous les éléments nécessaires à la survie et/ou à la croissance des microorganismes. Ce milieu réactionnel peut soit servir uniquement de milieu de révélation, soit servir de milieu de culture et de révélation. Dans le premier cas, la culture des microorganismes peut être effectuée avant ensemencement, et, dans le second cas, le milieu réactionnel constitue également le milieu de culture. Le milieu réactionnel peut être solide, semi-solide ou liquide. Par milieu solide, on entend par exemple un milieu gélifié. Préférentiellement, le milieu selon l'invention est un milieu gélifié. L'agar est l'agent gélifiant traditionnel en microbiologie pour la culture des microorganismes, mais il est possible d'utiliser d'autres agents gélifiants comme par exemple la gelrite, la gélatine ou l'agarose. Le milieu réactionnel selon l'invention peut contenir d'éventuels additifs comme par exemple : des peptones, un ou plusieurs facteurs de croissance, des hydrates de carbone, un ou plusieurs agents sélectifs, des tampons, un ou plusieurs gélifiants etc. Ce milieu réactionnel peut se présenter sous forme de liquide, de gel prêt à l'emploi, c'est-à-dire prêt à l'ensemencement en tube, flacon ou sur boite de Pétri.

D'une manière générale, le milieu réactionnel peut en sus contenir un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou chromogène. Pour une détection indirecte, le milieu réactionnel selon l'invention peut comporter en sus un indicateur de pH, sensible à la variation de pH induite par la consommation du substrat et révélant la croissance des microorganismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'hydroxycoumarine ou les dérivés de la résorufine. Ainsi, le substrat fluorescent de PC-PLC préférentiellement utilisé pour la mise en oeuvre du procédé selon l'invention correspond au 4-Méthyl-Umbelliferyl-Choline Phosphate (4 MU-CP).

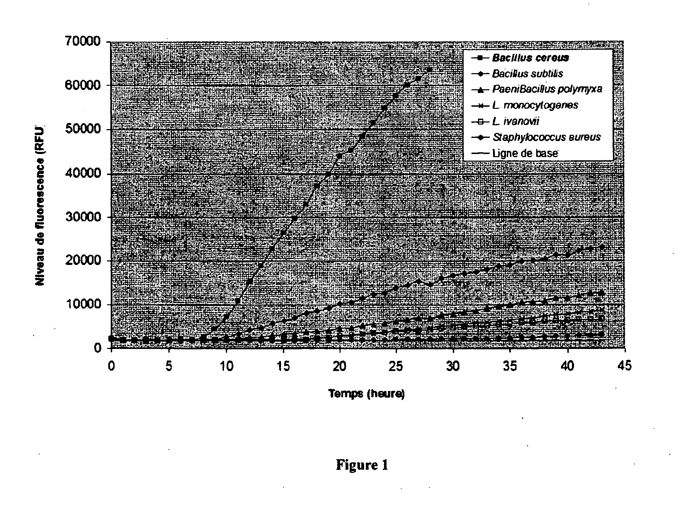

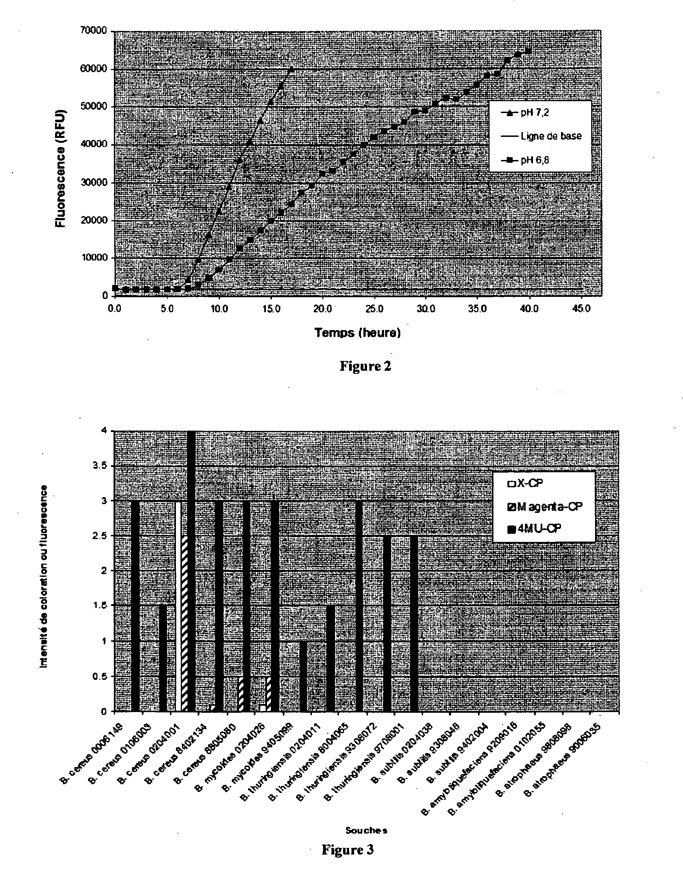

La compréhension du procédé selon l'invention sera favorisée par les exemples *infra* qui n'ont aucun caractère limitatif, en combinaison avec le dessin dans lequel :.
● la Figure 1 illustre des mesures cinétiques de l'activité PC-PLC de différentes bactéries à Gram positif.
● la Figure 2 illustre la mesure cinétique d'activité PC-PLC de la souche *Bacillus cereus* ATCC 7064 en fonction du pH du milieu, respectivement fixé à 7,2 ou 6,8.
● la Figure 3 illustre l'intensité de coloration ou de fluorescence obtenue en 24h. pour diverses souches de *Bacillus spp* en fonction du type de substrat utilisé.

### Exemple 1 : Etude de l'activité PC-PLC de Bacillus cereus versus celle observée pour d'autres bactéries à Gram positif (Figure 1 et Tableau 1)

Différentes souches pures du genre *Bacillus* ainsi que d'autres bactéries à Gram positif ont été testées en microplaque en présence du milieu ci-dessous. Une lecture d'apparition de la fluorescence dans chacun des différents puits de la microplaque a ensuite été effectuée en cinétique durant 44h d'incubation à 37°C.

### 1. MILIEU

Le milieu de composition suivante a été utilisé (composition en g/l), pH 7,2 :

| **Composés** | **Concentration en g/l** |
|---|---|
| Extrait de levure | 5 |
| Pyruvate de sodium | 2 |
| Glycérophosphate de magnésium | 1 |
| Tampon HEPES basique | 13,8 |
| Tampon HEPES acide | 11,92 |
| 4-Methylumbelliferyl choline-phosphate¹ | 0,4 |

| | |
|---|---|
| '4-Methylumbelliferyl Choline-phosphate (4MU-PC), Biosynth®, Réf. M-5528 | |

### 2. ESSAIS

Les puits de la microplaque sont inoculés avec 10 Unités Formant Colonies (UFC) de bactéries du groupe *Bacillus cereus* et 1 000 UFC pour les autres *Bacillus* non *cereus* et autres bactéries à Gram positif. La microplaque est ensuite incubée pendant 44h à 37°C dans un lecteur de microplaques afin d'évaluer l'activité PC-PLC de ces diverses souches sous forme de cinétique d'hydrolyse du substrat 4MU-CP, *i.e* de détecter et mesurer l'apparition de fluorescence. Le seuil de détection de l'appareil de mesure est fixé à 30 000 RFU (relative fluorescence unit).

### 3. RÉSULTATS ET INTERPRETATION

La Figure 1 montre une différence significative entre l'activité PC-PLC observée chez *Bacillus cereus* (signal positif dès 15 heures) et celle observée chez les autres bactéries à Gram positif (signal positif détectable uniquement après 40 heures d'incubation). Il n'est pas nécessaire d'ajouter au milieu un système inhibiteur complexe pour obtenir ce résultat.

Ces résultats ont été confirmés pour 10 souches appartenant au groupe *Bacillus cereus versus* 15 autres souches n'appartenant pas au groupe *Bacillus cereus.* Ces derniers résultats sont regroupés dans le Tableau 1.

La discrimination des souches du groupe *Bacillus cereus, versus* les autres bactéries à Gram positif, est alors possible sans cocktail inhibiteur complexe (présence uniquement d'un inhibiteur anti Gram négatif) dans une fenêtre de lecture comprise entre 6 et 30h.

**Tableau.1 : Niveau de fluorescence généré à 24 et 40h par l'hydrolyse du 4MU-PC par divers microorganismes. Seuil de détection: 30 000 RFU. Les bactéries appartenant au groupe Bacillus cereus sont indiquées en caractères gras.**

| **Espèces bactériennes** | **Niveaux de fluorescence en 24h (RFU)** | **Niveaux de fluorescence en 40h (RFU)** |
|---|---|---|
| ***Bacillus cereus* ATCC 7064** | **> 60000** | **> 60000** |
| ***Bacillus cereus* ATCC 6464** | **> 60000** | **> 60000** |
| ***Bacillus cereus* ATCC 9139** | **> 60000** | **> 60000** |
| ***Bacillus cereus* ATCC 10876** | **> 60000** | **> 60000** |
| ***Bacillus cereus* ATCC 33019** | **> 60000** | **> 60000** |
| ***Bacillus cereus* NCTC 11145** | **> 60000** | **> 60000** |
| ***Bacillus thuringiensis* 0240015** | **> 60000** | **> 60000** |
| ***Bacillus mycoides* ATCC 6463** | **> 60000** | **> 60000** |
| *Bacillus licheniformis* 93.08.043 | 2000 | 2000 |
| *Bacillus sphaericus* 8710054 | 20000 | > 60000 |
| *Bacillus circulans* ATCC 4513 | 10000 | 20000 |
| *Bacillus subtilis* ATCC 6051 | 10000 | 20000 |
| *Bacillus lentus* ATCC 10840 | 5000 | 10000 |
| *Bacillus pumilus* ATCC 7061 | 5000 | 10000 |
| *PaeniBacillus polymyxa* ATCC 21551 | 5000 | 10000 |
| *Bacillus megaterium* ATCC 14581 | 5000 | 5000 |
| *L. monocytogenes* ATCC 19118 | 4000 | 15000 |
| *L. monocytogenes* 0301902 | 5000 | 10000 |
| *L. ivanovii* ATCC 49954 | 2000 | 10000 |
| *L. ivanovii* 0002147 | 2000 | 10000 |
| *S. aureus* 8407603 | 2000 | 2000 |
| *S. aureus* 25923 | 2000 | 2000 |
| **Ligne de base** | **2000** | **2000** |

### Exemple 2 : Etude de l'activité PC-PLC de Bacillus cereus à pH 7,2 versus pH 6,8 (Figure 2)

La dynamique de l'activité PC-PLC de *Bacillus cereus* ATCC 7064 a été évaluée à pH 7,2 (milieu A) *versus* pH 6,8 (milieu B).

### 1. MILIEUX

Le milieu A est de composition suivante, pH 7,2 :

| **Composés** | **Concentration en g/l** |
|---|---|
| Extrait de levure | 5 |
| Pyruvate de sodium | 2 |
| Glycérophosphate de magnésium | 1 |
| Tampon HEPES basique | 13,8 |
| Tampon HEPES acide | 11,92 |
| 4-Methylumbelliferyl choline-phosphate | 0,4 |

Le milieu B est de composition suivante, pH 6,8 :

| **Composés** | **Concentration en g/l** |
|---|---|
| Extrait de levure | 5 |
| Pyruvate de sodium | 2 |
| Glycérophosphate de magnésium | 1 |
| Tampon PIPES Na | 16,22 |
| Tampon PIPES diNa | 17,32 |
| 4-Methylumbelliferyl choline-phosphate | 0,4 |

### 2. ESSAIS

Dix UFC de *Bacillus cereus* ATCC 7064 ont été inoculées dans les puits de la microplaque en présence du milieu A (pH 7,2) et du milieu B (pH 6,8). La microplaque est ensuite incubée pendant 44h à 37°C dans un lecteur de microplaques afin d'évaluer l'activité PC-PLC de la souche de *Bacillus cereus* ATCC 7064 aux deux pH étudiés.

### 3. RESULTATS ET INTERPRETATION

La dynamique d'activité PC-PLC de *Bacillus cereus* ATCC 7064 et les signaux RFU ainsi obtenus à pH 7,2 sont supérieurs. En effet, l'intensité d'émission de fluorescence de la 4MU augmente avec le pH du milieu (pH optimal d'émission = 10).

Considérant la croissance et l'activité PC-PLC de *Bacillus cereus,* le pH optimal du milieu est de 7,2. L'obtention de signaux supérieurs permet alors de diminuer le temps de détection, *i.e* de 10h dans ce cas précis si on considère le seuil de détection à 30 000 RFU.

*Nota Bene :* dans le cas d'une utilisation du 4MU-MIP (4-Methylumbelliferyl myo-inositol-1-phosphate, N-methyl-morpholine salt, Biosynth®, Réf. M-5717) pour différencier les bactéries du groupe *Bacillus cereus,* ce pH de 7,2 ne peut-être utilisé en raison d'une instabilité trop importante du substrat. Un pH de 6,8 est alors requis entraînant une augmentation du temps de détection.

### Exemple 3 : Etude des performances de différents substrats (chromogéniques versus fluorogéniques) pour révéler l'activité PC-PLC des bactéries du groupe Bacillus cereus versus Bacillus spp. (Figure 3)

Différentes souches du genre *Bacillus* ont été testées sur 10 milieux différents. La lecture des boîtes est ensuite effectuée après 24h et 48h d'incubation à 37°C.

Les substrats testés sont le 5-bromo-4-chloro-3-indoxyl-choline phosphate (X-CP), chromogène, le 5-bromo-6-chloro-3-indoxyl-choline phosphate (magenta-CP), chromogène, et le 4-méthylumbelliferone-choline phosphate (4MU-CP), fluorescent.

### 1. MILIEUX

Le milieu de composition suivante a été utilisé (composition en g/l) :
- extrait de levure : 5 g/l
- glycérophosphate de magnésium : 1 g/l
- agar: 13 g/l
- LiCl : 3 g/l
- MOPS : 12,6 g/l
- MOPS sodium salt : 20,78 g/l

Ce milieu a été réparti dans 10 flacons différents (T, 1, ..., 9) qui ont ensuite été stérilisés par un cycle d'autoclave 15min/121°C. Le milieu T sert de témoin de croissance. Des solutions-mères à 30g/l de 5-bromo-4-chloro-3-indoxyl-choline phosphate (X-CP), 5-bromo-6-chloro-3-indoxyl-choline phosphate (magenta-CP) et 4-méthylumbelliferone-choline phosphate (4MU-CP) ont été préparées dans de l'eau osmosée. Ensuite un volume correspondant à une concentration finale en X-CP respectivement de 100, 300 et 900mg/l a été ajouté dans les milieux en surfusion notés respectivement 1, 2 et 3. On répète la même opération pour les milieux 4, 5 et 6 et 7, 8 et 9 contenant respectivement 100, 300 et 900mg/l de magenta-CP et 100, 300 et 900mg/l de 4MU-CP. Ces milieux gélosés ont été coulés en boîtes de Petri.

### 2. ESSAIS

L'inoculation des différentes souches de *Bacillus* a été effectuée par isolement en trois cadrans à partir de suspensions à 0.5 McF (unités McFarland). Les boîtes ont ensuite été incubées pendant 48h à 37°C.

Les colonies formées ont été examinées visuellement après 24 et 48h d'incubation. La coloration ou fluorescence (lue sous lampe UV à 366 nm) de ces colonies ainsi que les intensités ont été notées.

### 3. RESULTATS

Les intensités de coloration et fluorescence sont lues sur une échelle relative allant de 0 (aucune coloration/fluorescence) à 4 (coloration/fluorescence très intense).

Les résultats sont illustrés sur la Figure 3 (expression de l'activité PC-PLC après 24h d'incubation sur différentes espèces de *Bacillus).* L'absence de barre indique que l'intensité de coloration ou de fluorescence mesurée n'est pas significativement différente du bruit de fond, donc qu'il s'agit d'un résultat négatif.

### 4. INTERPRETATION

L'utilisation du substrat fluorogénique de PC-PLC, le 4MU-CP, contrairement aux substrats chromogéniques (X-CP et magenta-CP), permet la détection et la discrimination des bactéries du groupe *Bacillus cereus* versus *Bacillus subtilis* avec une sensibilité et une spécificité de détection élevées (100%); notamment après 24h d'incubation à 37°C et sur l'ensemble des souches testées.

## Revendications

1. Procédé d'identification des bactéries du groupe *Bacillus cereus,* comprenant les étapes consistant à :
● mettre en contact, dans un conteneur, un échantillon susceptible de contenir des bactéries du groupe *Bacillus cereus,* un milieu réactionnel comprenant au moins un substrat fluorescent de PC-PLC et un inhibiteur des bactéries Gram négatives;
● incuber l'ensemble ;
● identifier les bactéries du groupe *Bacillus cereus* par détection de la réaction d'hydrolyse du substrat de PC-PLC,
**caractérisé en ce que** le pH du milieu réactionnel et le temps nécessaire à la détection de la réaction d'hydrolyse du substrat de PC-PLC mise en oeuvre, sont adaptés de telle façon que ladite réaction d'hydrolyse par les bactéries du groupe *Bacillus cereus* est détectée avant que l'hydrolyse du substrat de PC-PLC par les bactéries Gram positives autre que celles appartenant au groupe *Bacillus cereus* et potentiellement présentes dans l'échantillon, ne soit détectable.

2. Procédé selon la revendication 1 **caractérisé en ce que** le pH du milieu réactionnel est compris entre 6,8 et 8,0.

3. Procédé selon la revendication 1 ou 2 **caractérisé en ce que** le temps nécessaire à la détection de l'hydrolyse du substrat de PC-PLC est compris entre 6 et 30 heures.

4. Procédé selon l'une des revendications précédentes dans lequel le milieu réactionnel est sous forme liquide, gel ou solide.

5. Procédé selon l'une des revendications précédentes dans lequel le milieu réactionnel est un milieu de culture.

6. Procédé selon l'une des revendications précédentes comprenant une étape préalable d'enrichissement de l'échantillon.

7. Procédé selon l'une des revendications précédentes, dans lequel le milieu comprend en outre au moins un second substrat, chromogène ou fluorescent.

8. Procédé selon la revendication précédente, dans lequel le second substrat est un substrat de PI-PLC permettant de discriminer *Bacillus anthracis* de *Bacillus cereus, Bacillus thuringiensis, Bacillus weihenstephanensis, Bacillus mycoides* et *Bacillus pseudomycoides.*

9. Procédé selon l'une des revendications précédentes **caractérisé en ce qu'**un dénombrement des bactéries du groupe *Bacillus cereus* est également possible.

10. Procédé selon l'une des revendications précédentes dans lequel le conteneur est pris dans le groupe constitué par les microplaques, les microcupules, les microtubes, les capillaires ou les cartes multipuits.

11. Procédé selon l'une des revendications précédentes, dans lequel le substrat fluorescent de PC-PLC correspond au 4 MU-CP (4-méthyl-umbelliferyl-choline phosphate).

12. Procédé selon l'une des revendications précédentes, dans lequel les bactéries du groupe *Bacillus cereus* sont choisies parmi *Bacillus cereus, Bacillus anthracis, Bacillus thuringiensis, Bacillus mycoides, Bacillus pseudomycoides, Bacillus weihenstephanensis,* et les autres bactéries sont choisies parmi *Listeria monocytogenes, Listeria ivanovii, Staphylococcus* ou les autres espèces du genre *Bacillus spp.* telles *Bacillus subtilis, Bacillus sphaericus, Bacillus circulans, Bacillus lentus, Bacillus pumilus, Bacillus megaterium, Paenibacillus polymyxa.*

13. Kit de diagnostic permettant la mise en oeuvre du procédé selon l'une des revendications précédentes, et comprenant un milieu réactionnel sélectif ou non, ledit milieu comprenant au moins un inhibiteur des bactéries Gram négatives et un substrat fluorescent de PC-PLC.

14. Kit de diagnostic selon la revendication précédente, comprenant en outre un conteneur, pris dans le groupe constitué par les microplaques, les microcupules, les microtubes, les capillaires et les cartes multipuits.

15. Kit de diagnostic selon l'une des revendications 13 ou 14, dans lequel le substrat fluorescent de PC-PLC correspond au 4 MU-CP (4-méthyl-umbelliferyl-choline phosphate).

16. Kit de diagnostic selon l'une des revendications 13 à 15, dans lequel le milieu réactionnel comprend en outre au moins un second substrat, chromogène ou fluorescent.

17. Kit de diagnostic selon la revendication 16, dans lequel le second substrat est un substrat de PI-PLC permettant de discriminer *Bacillus anthracis* de *Bacillus cereus, Bacillus thuringiensis, Bacillus weihenstephanensis, Bacillus mycoides* et *Bacillus pseudomycoides.*

## Patentansprüche

1. Ein Verfahren zur Identifizierung von Bakterien der Gruppe *Bacillus cereus,* das die Schritte beinhaltet, die aus Folgendem bestehen:
● eine Probe, die Bakterien der Gruppe *Bacillus cereus* enthalten kann, ein Reaktionsmedium, das mindestens ein fluoreszierendes PC-PLC-Substrat und einen Inhibitor gramnegativer Bakterien beinhaltet, in einem Behältnis in Kontakt zu bringen;
● das Ganze zu inkubieren;
● die Bakterien der Gruppe *Bacillus cereus* durch Nachweis der Hydrolysereaktion des PC-PLC-Substrats zu identifizieren,
**dadurch gekennzeichnet, dass** der pH-Wert des Reaktionsmediums und der Zeitraum, der für den Nachweis der Hydrolysereaktion des verwendeten PC-PLC-Substrats notwendig ist, derart angepasst sind, dass die Hydrolysereaktion durch die Bakterien der Gruppe *Bacillus cereus* nachgewiesen wird, bevor die Hydrolyse des PC-PLC-Substrats durch andere grampositive Bakterien als diejenigen, die zur Gruppe *Bacillus cereus* gehören und in der Probe potenziell vorhanden sind, nachweisbar ist.

2. Verfahren gemäß Anspruch 1, **dadurch gekennzeichnet, dass** der pH-Wert des Reaktionsmediums zwischen 6,8 und 8,0 liegt.

3. Verfahren gemäß Anspruch 1 oder 2, **dadurch gekennzeichnet, dass** der Zeitraum, der für den Nachweis der Hydrolyse des PC-PLC-Substrats notwendig ist, zwischen 6 und 30 Stunden liegt.

4. Verfahren gemäß einem der vorhergehenden Ansprüche, wobei das Reaktionsmedium in flüssiger, Gel- oder fester Form vorliegt.

5. Verfahren gemäß einem der vorhergehenden Ansprüche, wobei das Reaktionsmedium ein Nährmedium ist.

6. Verfahren gemäß einem der vorhergehenden Ansprüche, das einen vorherigen Schritt zur Anreicherung der Probe beinhaltet.

7. Verfahren gemäß einem der vorhergehenden Ansprüche, wobei das Medium zudem mindestens ein zweites, chromogenes oder fluoreszierendes Substrat beinhaltet.

8. Verfahren gemäß vorhergehendem Anspruch, wobei das zweite Substrat ein PI-PLC-Substrat ist, das ermöglicht, *Bacillus anthracis* von *Bacillus cereus, Bacillus thuringiensis, Bacillus weihenstephanensis, Bacillus mycoides* und *Bacillus pseudomycoides* zu unterscheiden.

9. Verfahren gemäß einem der vorhergehenden Ansprüche, **dadurch gekennzeichnet, dass** eine Auszählung der Bakterien der Gruppe *Bacillus cereus* ebenfalls möglich ist.

10. Verfahren gemäß einem der vorhergehenden Ansprüche, wobei das Behältnis aus der Gruppe, bestehend aus Mikroplatten, Mikrobechern, Mikroröhrchen, Kapillaren oder Multi-Well-Karten, genommen wird.

11. Verfahren gemäß einem der vorhergehenden Ansprüche, wobei das fluoreszierende PC-PLC-Substrat 4 MU-CP (4-Methylumbelliferylcholinphosphat) entspricht.

12. Verfahren gemäß einem der vorhergehenden Ansprüche, wobei die Bakterien der Gruppe *Bacillus cereus* aus *Bacillus cereus, Bacillus anthracis, Bacillus thuringiensis, Bacillus mycoides, Bacillus pseudomycoides, Bacillus weihenstephanensis* und die anderen Bakterien aus *Listeria monocytogenes, Listeria ivanovii, Staphylococcus* oder den anderen Spezies der Gattung *Bacillus spp.* ausgewählt werden, wie *Bacillus subtilis, Bacillus sphaericus, Bacillus circulans, Bacillus lentus, Bacillus pumilus, Bacillus megaterium, Paenibacillus polymyxa.*

13. Ein Diagnosekit, das die Verwendung des Verfahrens gemäß einem der vorhergehenden Ansprüche ermöglicht und ein Reaktionsmedium, selektiv oder nicht, beinhaltet, wobei das Medium mindestens einen Inhibitor der gramnegativen Bakterien und ein fluoreszierendes PC-PLC-Substrat beinhaltet.

14. Diagnosekit gemäß dem vorhergehenden Anspruch, das zudem ein Behältnis beinhaltet, das aus der Gruppe, bestehend aus Mikroplatten, Mikrobechern, Mikroröhrchen, Kapillaren oder Multi-Well-Karten, genommen wird.

15. Diagnosekit gemäß einem der Ansprüche 13 oder 14, in dem das fluoreszierende PC-PLC-Substrat 4 MU-CP (4-Methylumbelliferylcholinphosphat) entspricht.

16. Diagnosekit gemäß einem der Ansprüche 13 bis 15, wobei das Reaktionsmedium zudem mindestens ein zweites, chromogenes oder fluoreszierendes Substrat beinhaltet.

17. Diagnosekit gemäß Anspruch 16, wobei das zweite Substrat ein PI-PLC-Substrat ist, das ermöglicht, *Bacillus anthracis* von *Bacillus cereus, Bacillus thuringiensis, Bacillus weihenstephanensis, Bacillus mycoides* und *Bacillus pseudomycoides* zu unterscheiden.

## Claims

1. A method for identifying bacteria of the *Bacillus cereus* group, comprising the steps consisting in:
● bringing a sample that may contain bacteria of the *Bacillus cereus* group, a reaction medium comprising at least one fluorescent PC-PLC substrate and an inhibitor of Gram-negative bacteria into contact, in a container;
● incubating all of the above together;
● identifying the bacteria of the *Bacillus cereus* group by detecting the PC-PLC substrate hydrolysis reaction,
**characterized in that** the pH of the reaction medium and the time necessary for detecting the PC-PLC substrate hydrolysis reaction carried out are adapted in such a way that said hydrolysis reaction by the bacteria of the *Bacillus cereus* group is detected before the hydrolysis of the PC-PLC substrate by the Grampositive bacteria other than those belonging to the *Bacillus cereus* group, and potentially present in the sample, is detectable.

2. The method as claimed in claim 1, **characterized in that** the pH of the reaction medium is between 6.8 and 8.0.

3. The method as claimed in claim 1 or 2, **characterized in that** the time necessary for detecting the PC-PLC substrate hydrolysis is between 6 and 30 hours.

4. The method as claimed in one of the preceding claims, in which the reaction medium is in liquid, gel or solid form.

5. The method as claimed in one of the preceding claims, in which the reaction medium is a culture medium.

6. The method as claimed in one of the preceding claims, comprising a prior step of enrichment of the sample.

7. The method as claimed in one of the preceding claims, in which the medium also comprises at least one second substrate, which is chromogenic or fluorescent.

8. The method as claimed in the preceding claim, in which the second substrate is a PI-PLC substrate which makes it possible to distinguish *Bacillus anthracis* from *Bacillus cereus, Bacillus thuringiensis, Bacillus weihenstephanensis, Bacillus mycoides* and *Bacillus pseudomycoides.*

9. The method as claimed in one of the preceding claims, **characterized in that** counting of the bacteria of the *Bacillus cereus* group is also possible.

10. The method as claimed in one of the preceding claims, in which the container is selected from the group consisting of microplates, microwells, microtubes, capillaries or multiwell cards.

11. The method as claimed in one of the preceding claims, in which the fluorescent PC-PLC substrate corresponds to 4 MU-CP (4-methylumbelliferyl choline phosphate).

12. The method as claimed in one of the preceding claims, in which the bacteria of the *Bacillus cereus* group are chosen from *Bacillus cereus, Bacillus anthracis, Bacillus thuringiensis, Bacillus mycoides, Bacillus pseudomycoides* and *Bacillus weihenstephanensis,* and the other bacteria are chosen from *Listeria monocytogenes, Listeria ivanovii, Staphylococcus* or the other species of the genus *Bacillus spp.,* such as *Bacillus subtilis, Bacillus sphaericus, Bacillus circulans, Bacillus lentus, Bacillus pumilus, Bacillus megaterium* or *Paenibacillus polymyxa.*

13. A diagnostic kit for carrying out the method as claimed in one of the preceding claims, and comprising a selective or nonselective reaction medium, said medium comprising at least one inhibitor of Gram-negative bacteria and a fluorescent PC-PLC substrate.

14. The diagnostic kit as claimed in the preceding claim, also comprising a container, selected from the group consisting of micropiates, microwells, microtubes, capillaries and multiwell cards.

15. The diagnostic kit as claimed in either of claims 13 or 14, in which the fluorescent PC-PLC substrate corresponds to 4 MU-CP (4-methylumbelliferyl choline phosphate).

16. The diagnostic kit as claimed in one of claims 13 to 15, in which the reaction medium also comprises at least one second substrate, which is chromogenic or fluorescent.

17. The diagnostic kit as claimed in claim 16, in which the second substrate is a Pl-PLC substrate which makes it possible to distinguish *Bacillus anthracis* from *Bacillus cereus, Bacillus thuringiensis, Bacillus weihenstephanensis, Bacillus*
*mycoides* and *Bacillus pseudomycoides.*
